(19) Europäisches Patentamt
European Patent Office
Office européen des brevets

(11) Veröffentlichungsnummer: **0 283 540 B1**

(12) **EUROPÄISCHE PATENTSCHRIFT**

(45) Veröffentlichungstag der Patentschrift: **18.09.91**

(51) Int. Cl.$^5$: **C07C 217/00**

(21) Anmeldenummer: **87104494.7**

(22) Anmeldetag: **26.03.87**

# (54) Verfahren zur Herstellung von 5-Hydroxydiprafenon und seiner Salze mit Säuren.

(43) Veröffentlichungstag der Anmeldung:
**28.09.88 Patentblatt 88/39**

(45) Bekanntmachung des Hinweises auf die Patenterteilung:
**18.09.91 Patentblatt 91/38**

(84) Benannte Vertragsstaaten:
**AT BE CH DE ES FR GB GR IT LI LU NL SE**

(56) Entgegenhaltungen:
**EP-A- 0 075 207**
**DE-A- 3 328 376**

(73) Patentinhaber: **Helopharm W. Petrik GmbH & Co.KG.**
**Waldstrasse 23-25**
**W-1000 Berlin 51(DE)**

(72) Erfinder: **Petrik, Gerd**
**Herthastrasse 11**
**W-1000 Berlin 33(DE)**
Erfinder: **Schubert, Klemens, Dr.**
**Im Schönower Park 1E**
**W-1000 Berlin 37(DE)**

(74) Vertreter: **Vossius & Partner**
**Siebertstrasse 4 P.O. Box 86 07 67**
**W-8000 München 86(DE)**

**EP 0 283 540 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein neues Verfahren zur Herstellung von 5-Hydroxydiprafenon und seiner Salze mit Säuren sowie ein wesentliches Zwischenprodukt für dieses Verfahren.

Aus der DE-OS 33 28 376 ist unter anderem das 5-Hydroxydiprafenon, d.h., das 2-[2'-Hydroxy-3'-(1,1-dimethylpropylamino) - propoxy]-5-hydroxy-β-phenylpropiophenon bekannt. Die Herstellung erfolgt zweckmäßig ausgehend vom entsprechend substituierten Acetophenon, d.h. 2,5-Dihydroxyacetophenon, das nach Verätherung der phenolischen Hydroxylgruppe in 5-Stellung mit Benzylbromid (Ausbeute ca. 72 % d.Th.) mit Benzaldehyd zum entsprechenden a,β-ungesättigten Keton, d.h. 5-Benzyloxy-2-hydroxybenzalacetophenon (Ausbeute ca. 57 % d. Th.) umgesetzt wird. Diese Verbindung wird in nahezu quantitativer Ausbeute mit Epichlorhydrin zum 2-(2',3'-Epoxypropoxy)-5-benzyloxybenzalacetophenon und sodann mit 1,1-Dimethylpropylamin zum 2-[2'-Hydroxy-3'-(1,1 -dimethylpropylamino) -propoxy] -5-benzyloxybenzalacetophenon umgesetzt. Anschließend wird durch katalytische Hydrierung in Gegenwart von Palladium-auf-Kohlenstoff (5 %) das 5-Hydroxy-diprafenon in einer Ausbeute von etwa 17 % d.Th. erhalten. Die Gesamtausbeute dieses mehrstufigen Verfahrens beträgt etwa 7 %. Das Verfahren ist für die Herstellung 5-Hydroxydiprafenon in großem Maßstab nicht zweckmäßig, da es von in schlechter Ausbeute herstellbarem und deshalb relativ teurem 2,5-Dihydroxyacetophenon ausgeht. Die Acylierung von Hydrochinon nach Friedel-Crafts verläuft nämlich bevorzugt unter Bildung der Phenolester.

Der Erfindung liegt somit die Aufgabe zugrunde, ein Verfahren zur Herstellung von 5-Hydroxydiprafenon zur Verfügung zu stellen, bei dem leicht zugängliche Ausgangsverbindungen verwendet werden können und das eine erheblich verbesserte Gesamtausbeute ergibt. Eine weitere Aufgabe der Erfindung ist es, ein wesentliches Zwischenprodukt für dieses Verfahren zur Verfügung zu stellen.

Die Erfindung beruht auf folgendem Befund:

Aus den billigen und leicht zugänglichen Ausgangsverbindungen Hydrochinon und Hydrozimtsäure (β-Phenylpropionsäure) kann durch Kondensation mit Zinkchlorid in ca. 86prozentiger Ausbeute das 2,5-Dihydroxy-β-phenylpropiophenon erhalten werden. Die 5-Hydroxygruppe dieser Verbindung wird nun für die nachfolgende Reaktion mit einer Schutzgruppe versehen, die später wieder leicht abgespalten werden kann. Die bevorzugte Schutzgruppe ist die Benzylgruppe. Anschließend wird die 2-Hydroxygruppe mit Epichlorhydrin unter milden Bedingungen veräthert. Vorzugsweise wird diese Umsetzung in Isopropanol als Lösungsmittel und unter Rückflußkochen durchgeführt. Die Ausbeute ist quantitativ. Das Produkt kann ohne weitere Reinigung in die nächste Stufe eingesetzt werden. Anschließend wird die erhaltene 2-(2',3'-Epcxypropoxy)-β-phenylpropiophenon-Verbindung mit geschützter Hydroxylgruppe in 5-Stellung mit 1,1-Dimethylpropylamin (tert.-Pentylamin) unter milden Bedingungen umgesetzt. Vorzugsweise wird diese Umsetzung (Öffnung des Epoxidrings) in Methanol als Lösungsmittel und unter Rückflußkochen durchgeführt. Es wird das 2-[2'-Hydroxy-3'-(1,1-dimethylpropylamino)-propoxy]-β-phenylpropiophenon mit geschützter Hydroxylgruppe in 5-Stellung erhalten. Aus dieser Verbindung läßt sich die Schutzgruppe in der letzten Stufe des Verfahrens leicht wieder abspalten. Wenn die Schutzgruppe die Benzylgruppe ist, erfolgt die Abspaltung durch katalytische Hydrierung in Gegenwart von Palladium-auf-Kohlenstoff als Katalysator. Vorzugsweise wird 10prozentiges Palladium-auf-Kohlenstoff verwendet und die Umsetzung in Gegenwart von Methanol bei Raumtemperatur durchgeführt. Die Ausbeute beträgt in dieser Stufe etwa 73 % d.Th., die Gesamtausbeute mindestens etwa 46 % d. Th.

Gegebenenfalls wird das erhaltene 5-Hydroxydiprafenon durch Umsetzen mit einer anorganischen oder organischen Säure in ein Salz überführt. Als anorganische oder organische Säuren kommen beispielsweise in Betracht: Salzsäure, Bromwasserstoffsäure, Phosphorsäure, Schwefelsäure, Oxalsäure, Maleinsäure, Fumarsäure, Milchsäure, Weinsäure, Äpfelsäure, Citronensäure, Salicylsäure, Adipinsäure und Benzoesäure.

Die Salze werden in der Regel in an sich bekannter Weise durch Mischen der freien Base oder deren Lösungen mit der entsprechenden Säure oder deren Lösungen in einem organischen Lösungsmittel, beispielsweise einem niederem Alkohol, wie Methanol, Äthanol oder Propanol, oder einem niederen Keton, wie Aceton, Methyläthylketon oder Methylisobutylketon, oder einem Äther, wie Diäthyläther, Tetrahydrofuran oder Dioxan, erhalten. Zur besseren Kristallabscheidung können auch Mischungen der genannten Lösungsmittel verwendet werden.

5-Hydroxydiprafenon und seine Salze haben antiarrhythmische Eigenschaften.

Das Beispiel erläutert eine bevorzugte Ausführungsform des erfindungsgemäßen Verfahrens.

## Beispiel

a) 2,5-Dihydroxy-β-phenylpropiophenon

16,5 g (120 mMol) wasserfreies Zinkchlorid wurden mit 15 g (100 mMol) Hydrozimtsäure geschmolzen. In die entstandene klare Schmelze wurden 11 g (100 mMol) Hydrochinon eingetragen. Diese Mischung wurde 1,5 h auf 160 °C erhitzt. Das Reaktionsgemisch wurde mit Wasser verdünnt und mit 10prozentiger Salzsäure gewaschen. Das nach dem Abtrennen der wäßrigen Phase verbleibende öl kristallisierte aus und das erhaltene Rohprodukt wurde aus Wasser umkristallisiert. Ausbeute 20,8 g (86 % d.Th.); F. 118 bis 119 °C.

b) 2-Hydroxy-5-benzyloxy-β-phenylpropiophenon

20,8 g (86 mMol) 2,5-Dihydroxy-β-phenylpropiophenon, 15,4 ml (130 mMol) Benzylbromid und 12,5 g (90 mMol) Kaliumcarbonat wurden mit 200 ml Aceton 3 h unter Rückfluß erhitzt. Nach dem Abdestillieren des Lösemittels wurde der verbleibende Rückstand in Äthylacetat aufgenommen und dreimal mit je 150 ml 2 N Natronlauge ausgeschüttelt. Danach wurde die organische Phase mit 150 ml 2 N Salzsäure ausgeschüttelt und dann mit Wasser neutral gewaschen. Die organische Phase wurde abgetrennt, über Natriumsulfat getrocknet, filtriert und unter vermindertem Druck zur Trockene eingeengt. Der verbleibende Rückstand wurde aus Äthanol/Aceton (2:1) umkristallisiert. Ausbeute: 25,2 g (88,3 % d.Th.); F. 65 - 67 °C.

c) 2-(2',3'-Epoxypropoxy)-5-benzyloxy-β-phenylpropiophenon

25,2 g (75,9 mMol) 2-Hydroxy-5-benzyloxy-β-phenylpropiophenon wurden mit 50 ml Epichlorhydrin, 3,4 g Natriumhydroxid und 50 ml Isopropanol 6 h unter Rückfluß erhitzt. Danach wurde der Ansatz heiß filtriert und das Filtrat unter vermindertem Druck zur Trockene eingeengt. Der Rückstand wurde ohne Reinigung in die nächste Stufe eingesezt. Ausbeute quantitativ.

d) 2-[2'-Hydroxy-3'-(1,1-dimethylpropylamino)-propoxy]-5-benzyloxy-β-phenylpropiophenon

20,7 g (75,9 mMol) 2-(2',3'-Epoxypropoxy)-5-benzyloxy-β-phenylpropiophenon wurden mit 50 ml tert.-Pentylamin in 300 ml Methanol 3 h unter Rückfluß erhitzt.Nach dem Einengen unter vermindertem Druck wurde der verbleibende Rückstand mit 200 ml Methanol erhitzt. Nach dem Abkühlen wurden 3,2 g eines Nebenproduktes abgesaugt. Das Filtrat wurde unter vermindertem Druck zur Trockene eingeengt. Der Rückstand wurde ohne Reinigung in die nächste Stufe eingesetzt. Ausbeute: 30,4 g (84,3 % d. Th.).

e) 5-Hydroxydiprafenon-hydrochlorid

30,4 g (64 mMol) 2-[2'-Hydroxy-3'-(1,1-dimethylpropylamino)-propoxy]-5-benzyloxy-β-phenylpropiophenon wurden in 250 ml Methanol gelöst und unter Zusatz von 1 g Pd/C (10 %) hydriert. Nach beendeter Wasserstoffaufnahme (Verbrauch: 1450 ml) wurde der Katalysator abfiltriert und das Lösungsmittel abdestilliert. Der verbliebene Rückstand wurde mit 100 ml 1 N Salzsäure aufgekocht. Beim Abkühlen bildeten sich Kristalle, die abgesaugt und aus Aceton/Äthanol (2:1) umkristallisiert wurden. Ausbeute: 19,8 g (73 % d.Th.; 46,8 mMol entspricht 46,8 % d. Th., bezogen auf Hydrochinon); F. 165 - 167 °C.

**Patentansprüche**

**Patentansprüche für folgende Vertragsstaaten : AT, BE, CH, DE, FR, GB, GR, IT, LI, LU, NL, SE**

1. Verfahren zur Herstellung von 5-Hydroxydiprafenon der Formel

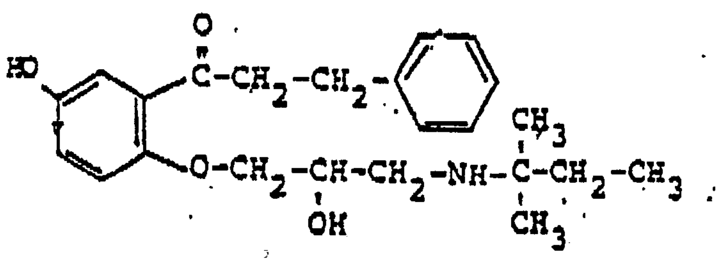

(I)

und seiner Salze mit Säuren, **dadurch gekennzeichnet,** daß man 2-[2'-Hydroxy-3'-(1,1-dimethylpropylamino)-propoxy]-5-benzyloxy-β-phenylpropiophenon in Gegenwart von Palladium-auf-Kohlenstoff als

Katalysator hydriert und gegebenenfalls das erhaltene 5-Hydroxydiprafenon durch Umsetzen mit einer Säure in ein Salz überführt.

2. Verfahren nach Anspruch 1, dadurch gekennzeichnet, daß das 2-[2'-Hydroxy-3'-(1,1-dimethylpropylamino)-propoxy]-5-benzyloxy-$\beta$-phenylpropiophenon hergestellt wird durch Umsetzen von Hydrochinon mit Hydrozimtsäure in Gegenwart von Zinkchlorid, Umsetzen des erhaltenen 2,5-Dihydroxy-$\beta$-phenylpropiophenons mit Benzylbromid zum 2-Hydroxy-5-benzyloxy-$\beta$-phenylpropiophenon, Umsetzen dieser Verbindung mit Epichlorhydrin zum 2-(2',3'-Epoxypropoxy)-5-benzyloxy-$\beta$-phenylpropiophenon und Umsetzen dieser Verbindung mit 1,1-Dimethylpropylamin.

3. 2-[2'-Hydroxy-3'-(1,1-dimethylpropylamino)-propoxy]-5-benzyloxy-$\beta$-phenylpropiophenon.

**Patentansprüche für folgenden Vertragsstaat : ES**

1. Verfahren zur Herstellung von 5-Hydroxydiprafenon der Formel

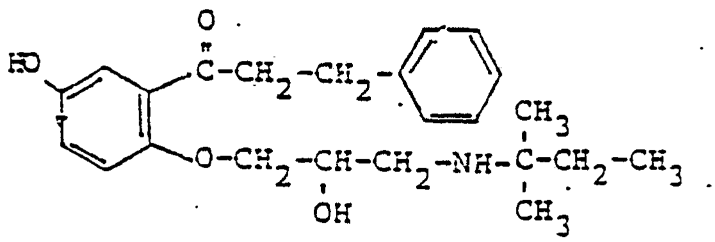

(I)

und seiner Salze mit Säuren, **dadurch gekennzeichnet,** daß man 2-[2'-Hydroxy-3'-(1,1-dimethylpropylamino)-propoxy]-5-benzyloxy-$\beta$-phenylpropiophenon in Gegenwart von Palladium-auf-Kohlenstoff als Katalysator hydriert und gegebenenfalls das erhaltene 5-Hydroxydiprafenon durch Umsetzen mit einer Säure in ein Salz überführt.

2. Verfahren nach Anspruch 1, dadurch gekennzeichnet, daß das 2-[2'-Hydroxy-3'-(1,1-dimethylpropylamino)-propoxy]-5-benzyloxy-$\beta$-phenylpropiophenon hergestellt wird durch Umsetzen von Hydrochinon mit Hydrozimtsäure in Gegenwart von Zinkchlorid, Umsetzen des erhaltenen 2,5-Dihydroxy-$\beta$-phenylpropiophenons mit Benzylbromid zum 2-Hydroxy-5-benzyloxy-$\beta$-phenylpropiophenon, Umsetzen dieser Verbindung mit Epichlorhydrin zum 2-(2',3'-Epoxypropoxy)-5-benzyloxy-$\beta$-phenylpropiophenon und Umsetzen dieser Verbindung mit 1,1-Dimethylpropylamin.

3. Verfahren zur Herstellung von 2-[2'-Hydroxy-3'-(1,1 -dimethylpropylamino)-propoxy]-5-benzyloxy-$\beta$-phenylpropiophenon, dadurch gekennzeichnet, daß Hydrochinon mit Hydrozimtsäure in Gegenwart von Zinkchlorid umgesetzt wird, das erhaltene 2,5-Dihydroxy-$\beta$-phenylpropiophenon mit Benzylbromid zum 2-Hydroxy-5-benzyloxy-$\beta$-phenylpropiophenonumgesetzt wird, diese Verbindung mit Epichlorhydrin zum 2-(2',3'-Epoxypropoxy)-5-benzyloxy-$\beta$-phenylpropiophenon umgesetzt wird, und diese Verbindung mit 1,1-Dimethylpropylamin umgesetzt wird.

**Claims**

**Claims for the following Contracting States : AT, BE, CH, DE, FR, GB, GR, IT, LI, LU, NL, SE**

1. Process for the preparation of 5-hydroxydipraphenone having the formula

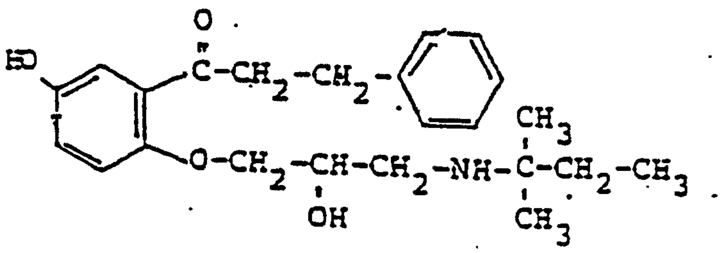 (I)

and its salts with acids, characterised in that 2-[2'-hydroxy-3'-(1 ,1-dimethylpropylamino)-propoxy]-5-benzyloxy-β-phenylpropiophenone is hydrogenated in the presence of palladium-on-carbon as a catalyst and optionally the 5-hydroxydipraphenone obtained is converted into a salt by reaction with an acid.

2. Process according to claim 1, characterised in that the 2-[2'-hydroxy-3'-(1,1-dimethylpropylamino)-propoxy]-5-benzyloxy-β-phenylpropiophenone is produced by reacting hydroquinone with hydrocinnamic acid in the presence of zinc chloride, reacting the 2,5-dihydroxy-β-phenylpropiophenone obtained with benzyl bromide to form 2-hydroxy-5-benzyloxy-β-phenylpropiophenone, reacting this compound with epichlorohydrin to form 2-(2',3'-epoxypropoxy)-5-benzyloxy-β-phenylpropiophenone and reacting this compound with 1,1-dimethylpropylamine.

3. 2-[2'-hydroxy-3'(1,1-dimethylpropylamino)-propoxy]-5-benzyloxy-β-phenylpropiophenone.

**Claims for the following Contracting State : ES**

1. Process for the preparation of 5-hydroxydipraphenone having the formula

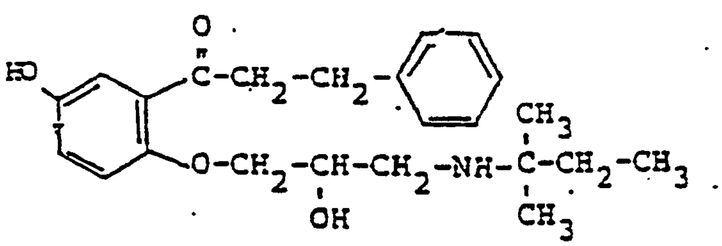 (I)

and its salts with acids, characterised in that 2-[2'-hydroxy-3'-(1,1-dimethylpropylamino)-propoxy]-5-benzyloxy-β-phenylpropiophenone is hydrogenated in the presence of palladium-on-carbon as a catalyst and optionally the 5-hydroxydipraphenone obtained is converted into a salt by reaction with an acid.

2. Process according to claim 1, characterised in that the 2-[2'-hydroxy-3'-(1,1-dimethylpropylamino)-propoxy]-5-benzyloxy-β-phenylpropiophenone is produced by reacting hydroquinone with hydrocinnamic acid in the presence of zinc chloride, reacting the 2,5-dihydroxy-β-phenylpropiophenone obtained with benzyl bromide to form 2-hydroxy-5-benzyloxy-β-phenylpropiophenone, reacting this compound with epichlorohydrin to form 2-(2',3'-epoxypropoxy)-5-benzyloxy-β-phenylpropiophenone and reacting this compound with 1,1-dimethylpropylamine.

3. Process for the preparation of 2-[2'-hydroxy-3'-(1,1-dimethylpropylamino)-propoxy]-5-benzyloxy-β-phenylpropiophenone, characterised in that hydroquinone is reacted with hydrocinnamic acid in the presence of zinc chloride, the 2,5-dihydroxy-β-phenylpropiophenone obtained is reacted with benzyl bromide to form 2-hydroxy-5-benzyloxy-β-phenylpropiophenone, this compound is reacted with epichlorohydrin to form 2-(2',3'-epoxypropoxy)-5-benzyloxy-β-phenylpropiophenone, and this compound is reacted with 1,1-dimethylpropylamine.

**Revendications**

**Revendications pour les Etats contractants suivants : AT, BE, CH, DE, FR, GB, GR, IT, LI, LU, NL, SE**

1. Procédé de préparation de 5-hydroxydipraténone de la formule

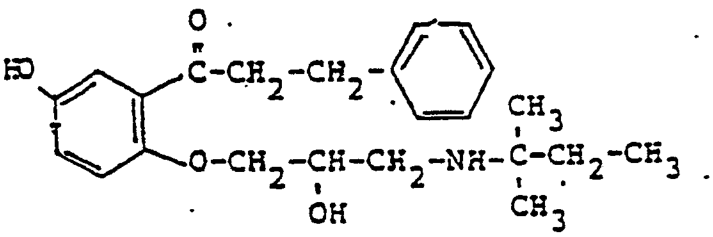

(I)

et de ses sels avec des acides, caractérise en ce qu'on hydrogène de la 2-[2'-hydroxy-3'(1,1-diméthylpropylamino)-propoxy]-5-benzyloxy-β-phénylpropiophénone en présence de palladium sur carbone, comme catalyseur, et en ce qu'éventuellement on convertit en un sel la 5-hydroxydipraténone obtenue par réaction avec un acide.

2. Procédé suivant la revendication 1, caractérisé en ce qu'on prépare la 2-[2'-hydroxy-3'(1,1-diméthylpropylamino)-propoxy]-5-benzyloxy-β-phénylpropiophénone par réaction d'hydroquinone avec de l'acide hydrocinnamique en présence de chlorure de zinc, réaction de la 2,5-dihydroxy-β-phénylpropiophénone obtenue avec du bromure de benzyle pour former de la 2-hydroxy-5-benzyloxy-β-phényl-propiophénone, réaction de ce composé avec de l'épichlorhydrine pour former de la 2-(2',3'-époxypropoxy)-5-benzyloxy-β-phénylpropio-phénone et réaction de ce composé avec de la 1,1-diméthylpropylamine.

3. 2-[2'-hydroxy-3'-(1,1-diméthylpropylamino)-propoxy]-5-benzyloxy-β-phénylpropiophénone.

**Revendications pour l'Etat contractant suivant : ES**

1. Procédé de préparation de 5-hydroxydipraténone de la formule

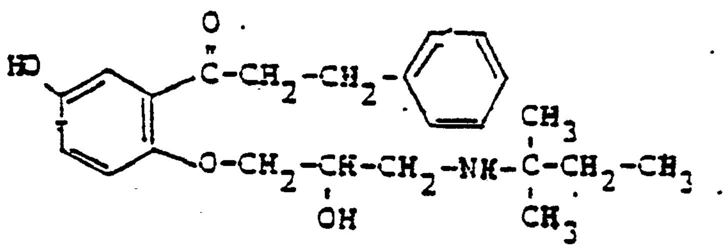

(I)

et de ses sels avec des acides, caractérisé en ce qu'on hydrogène de la 2-[2'-hydroxy-3'-(1,1-diméthylpropylamino)-propoxy]-5-benzyloxy-β-phénylpropiophénone en présence de palladium sur carbone, comme catalyseur, et en ce qu'éventuellement on convertit en un sel la 5-hydroxydipraténone obtenue par réaction avec un acide.

2. Procédé suivant la revendication 1, caractérisé en ce qu'on prépare la 2-[2'-hydroxy-3'-(1,1-diméthylpropylamino)-propoxy]-5-benzyloxy-β-phénylpropiophénone par réaction d'hydroquinone avec de l'acide hydrocinnamique en présence de chlorure de zinc, réaction de la 2,5 -dihydroxy-β-phénylpropiophénone obtenue avec du bromure de benzyle pour former de la 2-hydroxy-5-benzyloxy-β-phénylpropiophénone, réaction de ce compose avec de l'épicblorhydrine pour former de la 2-(2',3'-époxypropoxy)-5-benzyloxy-β-phénylpropiophénone et réaction de ce composé avec de la 1,1-diméthylpropylamine.

3. Procédé de préparation de 2-[2'-hydroxy-3'-(1,1-diméthylpropylamino)-propoxy]-5-benzyloxy-β-phénylpropiophénone, caractérisé en ce qu'on fait réagir de l'hydroquinone avec de l'acide hydrocinnamique en présence de chlorure de zinc, en ce qu'on fait réagir la 2,5-dihydroxy-β-phénylpropiophénone

obtenue avec du bromure de benzyle pour former de la 2-hydroxy-5-benzyloxy-β-phénylpropiophénone, en ce qu'on fait réagir ce composé avec de l'épichlorhydrine pour former de la 2-(2',3'-époxypropoxy)-5-benzyloxy-β-phénylpropiophénone et en ce qu'on fait réagir ce composé avec de la 1,1-diméthylpropylamine.